Europäisches Patentamt

European Patent Office

Office européen des brevets

(11) Publication number: **0 445 650 A2**

# EUROPEAN PATENT APPLICATION

(21) Application number: **91102957.7**

(22) Date of filing: **27.02.91**

(51) Int. Cl.5: **G01N 33/569**, //G01N33/543

(30) Priority: **05.03.90 US 488196**

(43) Date of publication of application:
**11.09.91 Bulletin 91/37**

(84) Designated Contracting States:
**DE ES FR IT**

(71) Applicant: **ABBOTT LABORATORIES**
**CHAD-0377, AP6D/2, One Abbott Park Road**
**Abbott Park, Illinois 60064-3500(US)**

(72) Inventor: **Stramer, Susan L.**
**9257 N. Hamlin Avenue**
**Evanston, Illinois 60203(US)**
Inventor: **Allain, Jean-Pierre**
**825 Morningside Drive**
**Lake Forest, Illinois 60045(US)**

(74) Representative: **Modiano, Guido et al**
**MODIANO, JOSIF, PISANTY & STAUB**
**Modiano & Associati Via Meravigli, 16**
**I-20123 Milano(IT)**

(54) **Detection of anti-hiv antibodies.**

(57) A method for the detection of anti-HIV antibodies in urine is disclosed. The method involves the steps of (a) collecting a urine sample from an individual, (b) diluting the urine sample with a diluent, and (c) assaying the diluted sample for the presence of anti-HIV antibodies.

EP 0 445 650 A2

BACKGROUND OF THE INVENTION

1. Field of the Invention

The present invention relates generally to a method for detecting anti-HIV antibodies in dilute urine samples.

2. Description of Related Art

Available data indicate that the acquired immunodeficiency syndrome (AIDS) is caused by a virus transmitted by sexual contact, by exposure to blood or blood products, or by transmission of the virus from an infected mother to her fetus or child during the perinatal period. Des Jarlis et al., MMWR (1984) 33:377-79. HIV-1 has been isolated from symptomatic individuals with AIDS and AIDS related complex (ARC), and from asymptomatic or healthy individuals who are at high risk for AIDS. Barre-Sinoussi et al., Science - (1983) 220:868-71. The incidence of anti-HIV antibodies in the blood of these individuals is high. A variety of serological tests have been developed to determine an anti-HIV antibody titer in the blood of AIDS patients, ARC patients and healthy asymptomatic individuals. The detection of anti-HIV antibodies by these tests are indicative of previous exposure to HIV. In populations at low risk for AIDS, such as volunteer blood donors, the expected incidence of seropositive individuals is low. In these populations, the percent of initially reactive individuals is typically 0.5% or less. Further, the numbers of initially reactive samples that repeat on duplicate retests is even lower, typically less than 0.1% with between 0.01% and 0.02% confirming by western blot testing.

The detection of anti-HIV antibodies to in urine has been reported using enzyme immunoassay technology. However, methods to date have used concentrated or undiluted urine for testing. Problems encountered include urine's variable antibody titer and limitations in the sensitivity of the particular immunoassays employed.

U.S. Patent No. 4,865,966 discloses such a method for determining an antibody titer to HIV in urine. This method involves concentrating the urine sample, preferably a 40 tb 100 mL sample, at least 20-fold relative to its initial (void) volume. The concentrating step can typically take between 60 and 90 minutes to complete. The total time required to test a urine sample for the presence of anti-HIV antibodies is longer than the time required to test a blood sample for the presence of anti-HIV antibodies, due to the additional step of concentrating the urine.

In order to avoid the inconvenience of the urine-concentrating step, some investigators have used unconcentrated urine directly in an immunoassay. For example, Cao et al., AIDS Res. Hum. Retroviruses - (1989) 5:311-19, disclose a method for detecting the presence of anti-HIV antibodies in unconcentrated urine specimens by utilizing 200 microliters ($\mu$L) of unconcentrated urine. However, the probability of nonspecific reactions would be expected to increase significantly in immunoassays that employ unconcentrated urine due to the sensitivity of immunoassays designed to detect anti-HIV antibodies.

A method using a large volume of unconcentrated urine may not yield reproducible results because nonspecific reactions are commonly associated with the use of large sample volumes in viral-based immunoassays. Nonspecific reactivity necessitates that all samples found to be reactive be investigated further in a manner that improves the predicatability that HIV-1 antibody, in fact, is present.

Furthermore, the unconcentrated urine, by contacting the antigens on the solid support, can affect antigen activity in the immunoassay because of the variability of urine chemistry (e.g., salt concentration, protein concentration, and pH).

There is a need, therefore, for an improved method for detecting the presence of antibodies in urine to increase the specificity and sensitivity of the test results.

**SUMMARY OF THE INVENTION**

The present invention provides a method of detecting anti-HIV antibodies in urine which comprises the steps of (a) collecting a urine sample from an individual, (b) diluting the urine sample with a diluent, and (c) assaying the diluted sample for the presence of anti-HIV antibodies.

**DETAILED DESCRIPTION OF THE INVENTION**

The present invention provides a method of detecting anti-HIV antibodies which comprises the steps of (a) collecting a urine sample from an individual, (b) diluting the urine sample with a diluent, and (c) assaying the diluted sample for the presence of anti-HIV antibodies.

It has been discovered that it is especially advantageous to dilute the collected urine sample in a diluent (hereinafter defined) to both simplify the immunoassay method and to improve the detection of the presence of anti-HIV antibodies. Thus, the method of the invention is a significant improvement over prior art immunoassay methods for detecting anti-HIV antibodies in urine.

In accordance with the present invention, a "diluent" is defined as an aqueous solution of buffer(s) and salt(s) as well understood in the art and illustrated infra. While a preferred buffer is Tris[hydroxymethyl]-aminomethane, commercially available under the trade designation Tris from Sigma Chemical Co., St. Louis, MO, suitable buffers include, but are not limited to, buff ers such as phosphate, HEPES (N-[2-hydroxyethyl]piperazine-N'-[2-ethanesulfonic acid]), PIPES (piperazine-N,N'-bis[2-ethanesulfonic acid]), CAPS (3-[cyclohexylamino]-1-propanesulfonic acid) and MOPES (3-[N-morpholino]propanesulfonic acid). Suitable salts include sodium chloride (NaCl) and salts such as phosphate salts and sulfate salts.

In addition, various animal sera, detergents, blocking agents and other components can be added to improve specificity. For example, animal serum proteins such as bovine serum, bovine serum albumin, fetal calf serum and goat serum can be added in concentrations ranging from about 0.5% v/v to about 50% v/v. Biological detergents such as polyoxyethylenesorbitan, commercially available as Tween 20, polyoxyethylene ether, commercially available as Triton® X-100, Nonidet P-40 (an octylphenol-ethylene oxide condensate), sodium dodecyl sulfate (SDS) or N-lauroylsarcosine (N-dodecanoyl-N-methylglycine) can be added in concentrations ranging from about 0.01% v/v to about 5% v/v. Chelators such as ethylenediaminetetraacetic acid (EDTA) and ethylene glycolbis($\beta$-aminoethylether) N,N,N',N'-tetraacetic acid (EGTA) can be added in concentrations ranging from about 2 mM to about 20 mM.

In order to neutralize the nonspecific reactions due to the other proteins contained in the viral lysates or recombinant proteins that comprise the antigen solutions which are employed on the solid support against which the diluted urine sample is reacted, a lymphocyte lysate solution, for example, human T-lymphocyte solution, or a host cell lysate solution such as an E. coli lysate solution, can be added in concentrations of from about 0.01% to about 10%. Preservatives such as sodium azide can also be added.

The urine sample can be diluted in a ratio of urine to diluent of from about 4 parts urine to 1 part diluent to about 1 part urine to 4 parts diluent. Preferably, the urine is diluted at a 1:1 ratio. Alternatively, the diluent can be added as a concentrate to the undiluted urine sample.

Acceptable immunoassay methods for the practice of the invention include those assays that employ HIV-1 antigens derived from native virus grown in human cell lines, recombinant proteins grown in prokaryotic or eukaryotic expression systems and synthetic peptides. Various immunoassay technologies, which involve the formation of antigen-antibody complexes, are readily applicable to the method of the invention. Such assays include enzyme-linked immunosorbent assays (ELISA) or other assays that employ an antibody "sandwich" assay technique such as western blot and agglutination. Competitive assays also can be employed, wherein antibody present in the urine sample competes with labelled antibody for binding to HIV-1 antigens. Solid supports which can be used in the immunoassays of the invention include wells of reaction trays, test tubes, beads, strips, membranes, microparticles or other solid supports which are known to those skilled in the art. The antigen-antibody complexes thus formed can be detected according to well-known methods wherein enzymes, radiolabels or other detectable substances are attached to antibodies or antigens to detect the antigen-antibody complexes.

In a preferred embodiment of the present invention, the diluted sample is assayed for the presence of anti-HIV antibodies by reacting the diluted sample with viral HIV antigens bound to a solid phase. The viral antigens bound to the solid phase comprise a mixture of HIV-1 viral lysate and purified HIV-1 envelope and core proteins. The concentration is determined by the purity of the antigens and their specific activity in an immunoassay. Typical concentrations of the affinity-purified envelope proteins on the solid phase range from about 0.0010 to about 0.010 micrograms per unit test, preferably from about 0.0015 to about 0.0025 micrograms per unit test. Similarly, typical concentrations of the affinity-purified core proteins on the solid phase range from about 0.0010 to about 0.10 micrograms per unit test, preferably from about 0.005 to about 0.010 micrograms per unit test. The method is described in more detail hereinbelow.

One of ordinary skill in the art can utilize the methods disclosed herein to develop a method to detect antibodies to any other human immunodeficiency virus, such as HIV-2.

## METHODS

Antigen Purification

A) Viral

H9 cells infected with HTLV-III(b) were harvested at their peak log phase of growth (approximately 7-10 days). The cells were concentrated by ultrafiltration and then pelleted by centrifuging at 20,000 RPM in about 8 hours (or, alternatively, 25,000 RPM for 3 hours or 43,000 RPM for 2 hours). The pelleted cells were resuspended in a solution containing 1 mM Tris, 15 mM NaCl and 0:1 mM EDTA, pH 7.5 (TNE).

The resuspended pellet was clarified by centrifuging at 10,000 RPM for approximately 15 minutes. The supernatant was decanted, and the pellet was resuspended in TNE and centrifuged again. The supernatants were combined and centrifuged again.

Virus contained in the supernatant obtained as described above was pelleted through a 20% sucrose pad by layering a 20% sucrose solution under the supernatant in a centrifuge tube and centrifuging at 35,000 RPM for 4 hours at 2-8° C (or, alternatively, 20,000 RPM for 16-22 hours or 43,000 RPM for 2 hours 45 minutes). The pellets were resuspended in TNE.

The resuspended virus was then banded by equilibrium sedimentation in a sucrose gradient according to procedures well known to those skilled in the art (see for example, the method of Sundquist, *Archives of Virology* (1981) 68:115-127). Briefly, the resuspended virus was layered on top of a sucrose gradient prepared as follows. The gradient was prepared in a centrifuge tube by underlaying a 45% sucrose solution to a 30% sucrose solution, overlaying the resuspended virus on the gradient and centrifuging at 25,000 RPM overnight in a swinging bucket rotor (e.g., SW-25). The gradient was fractionated and those fractions containing protein showing p41 activity were pooled. The banded virus was diluted in TNE and pelleted by centrifuging at 35,000 RPM for 4 hours at 2 8° C (or, alternatively, 20,000 RPM for 16-22 hours or 43,000 RPM for 2 hours 45 minutes). The pelleted virus was resuspended in TNE, sonicated in a solution containing 0.5 M NaCl and 0.5% Triton X-100, and clarified by centrifuging at 35,0000 RPM for 4 hours (or 43,000 RPM for 2 hours 40 minutes). The supernatant was decanted to provide purified viral lysate.

The p24 protein was isolated from the purified viral lysate by immunoaffinity chromatography using a monoclonal antibody. (General methods of monoclonal antibody production are reviewed in Monoclonal Hybridoma Antibodies: Techniques and Applications, ed. J.G.R. Hurrell [CRC Press, Inc.,1982]). Briefly, sepharose gel, for example Sepharose® 4B(Pharmacia Fine Chemicals Inc., Uppsala, Sweden), was first coupled with a monoclonal antibody specific for HIV-1 p24, cross-linked with glutaraldehyde at 0.05% and washed with a solution containing 0.01 M Tris, 0.001 M EDTA, 0.75 M NaCl, and 0.5% Triton X-100, pH 8.1 (wash solution). The washed gel was then mixed with the purified viral lysate (2 mg antigen/ml gel) and tumbled on a rotator for 12-24 hours at 2-8° C. The lysate-gel mixture was then poured into a column, washed with 5 column volumes of wash solution, and the p24 was eluted with 4.0 M guanidine HCL. The fractions containing protein were pooled, and the eluted p24 was dialyzed overnight in a 0.017 M phosphate solution containing 0.145 M NaCl, pH 7.2.

A protein assay was conducted using Bio-Rad reagents (Bio-Rad Laboratories, Richmond, CA) according to the manufacturer's procedures, to determine protein concentration, and corrected for absolute p24 protein concentration by the use of scanning densitometry. By these procedures, preparations of antigen with greater than 95% HIV p24 were routinely made.

Similarly, the gp41 protein was isolated from the purified viral lysate by immunoaffinity chromatography using a monoclonal antibody. Briefly, sepharose gel, for example Sepharose® 4B (Pharmacia), was first coupled with a monoclonal antibody specific for HIV-1 gp41, cross-linked with glutaraldehyde at 0.125% and washed with the wash solution previously described. The washed gel was then mixed with the purified viral lysate (4 mg antigen/ml gel) and tumbled on a rotor for 12-24 hours at 2-8° C. The lysate-gel mixture was then poured into a column, washed with 5 column volumes of wash solution, and the gp41 was eluted with 5.7 M sodium iodide. The fractions containing activity were pooled, and the eluted gp41 was dialyzed overnight in a 0.017 M phosphate solution containing 0.145 M NaCl, pH 7.2.

A radioimmunoassay using a monoclonal antibody specific to gp41 was used to determine antigen concentration. By PAGE-SDS and scanning densitometry, preparations of antigen with greater than 85% HIV gp41 were routinely made.

B) Recombinant

The HIV genome expressing the core and envelope proteins of HIV-1 was cloned and the proteins were expressed in E. coli according to the methods disclosed in U.S. Patent Application Serial No. 020,282, filed Feb. 27, 1987, which is incorporated herein by reference.

Bead Coating

A) Viral

Polystyrene beads were incubated for approximately 1 hour at about 40° C with a solution of the affinity-purified proteins gp41 and p24 described previously, at a concentration from about 0.0015 to about 0.0025 micrograms per bead and from about 0.0050 to about 0.010 micrograms per bead

($\mu$g/bead), respectively and then, diluted in phosphate buffered saline (PBS). After washing the beads with PBS for approximately 1 hour at about 40°C, the beads were incubated with lysed HIV at a concentration of approximately 0.0010 to 0.010 $\mu$g/bead. The beads were again washed with PBS, incubated with a blocking solution comprising 0.5-2.0% bovine albumin and Tween 20 for approximately 1 hour at about 40°C. A final overcoat solution with a sucrose concentration of from about 5% to about 10% diluted in PBS was applied. The beads were then dried.

B) Recombinant

The recombinant beads were coated according to the method described above for the viral coated beads with the following modifications. The antigen coating solution comprised she recombinant core and envelope proteins described previously, at a concentration from about 0.005 to about 0.05 micrograms per bead and from about 0.004 to about 0.012 micrograms per bead ($\mu$g/bead), respectively, diluted in phosphate buffered saline (PBS). The polystyrene beads were incubated with the antigen coating solution for approximately 1.5 hours.

Although the following specific examples illustrate several preferred embodiments of the present invention, they are not to be construed as limiting the scope of the invention in any manner.

EXAMPLE 1

HIV Antibody Detection in Random Population

A number of insurance applicants selected at random was tested for the presence of anti-HIV antibodies in both serum and urine samples. A commercially available immunoassay was utilized for the detection of anti-HIV-1 antibodies in serum (Genetic Systems, Seattle, WA). This assay employed whole viral lysate as the source of its antigen. A commercially available immunoassay for the detection of anti-HIV-1 antibodies in serum (Abbott Laboratories, Abbott Park, IL) was utilized for the detection of antibodies in urine. The antigens in this assay comprised a mixture of affinity-purified envelope and core viral proteins and whole viral lysate. The urine samples were diluted 1:1 with diluent. The diluent utilized comprised a pH 6.8 buffer of 10 millimolar (mM) sodium phosphate, 50 mM Tris, 100 mM NaCl, 10% fetal calf serum, 20% goat serum, 0.2% Tween® 20, 0.1% Triton® X-100, 10 mM EGTA, 5.6 mM EDTA, 0.01% human T-lymphocyte solution and 0.1% $NaN_3$.

Serum samples were tested for the presence of anti-HIV-1 antibodies as described hereinbefore. Serum samples wherein the initial test results were reactive by the method described hereabove, were further tested by western blot (Du Pont Co., Wilmington, DE) to confirm the results. If at least two of the three HIV gene products, gag, env and pol were present, the sample was deemed to be confirmed as positive.

Urine samples from the same individual as the serum samples having negative results were then assayed by the urine immunoassay method of the present invention. The mean and standard deviation of this negative urine population were calculated. In one method, five times the standard deviation (5SD) was used to determine the assay cutoff value, i.e., the absorbance value at which all samples must equal or exceed to be reactive in the assay. This cutoff value was compared to a second method for determining the cutoff value. In this second method, a ratio (S/N) of the urine sample absorbance (S) to the mean serum negative control (supplied by the manufacturer) absorbance (N) was determined for each urine sample. For both determinations, a positive result was defined as a ratio of urine sample absorbance divided by either cutoff value that was greater than or equal to 1.00.

The method of the present invention for detecting the presence of anti-HIV antibodies in urine was compared against two other prior art methods, one employing recombinant HIV proteins on the solid phase (Abbott Laboratories), the other employing whole viral lysate (Genetic Systems). The former assay utilized 400 $\mu$L of urine and the latter assay utilized 100 $\mu$L of urine. As summarized in Table 1, the specificity of the method of the present invention was consistently better than the specificity of both prior art anti-HIV antibody detection methods. The specificity of the assay is defined as the percent of those samples classified as negative (based on serum HIV antibody testing) that tested negative in any of the urine assay methods. As shown in Table 1, the sensitivity of the method of the present invention, using the S/N cutoff value, was 97.5%, compared to 93.6% for the 400 $\mu$L urine method and 15.7% for the 100 $\mu$L urine method. Similarly, using the 5SD cutoff value, the sensitivity of the diluted urine method was 98.8%, compared to 97.9% for the 400 $\mu$L urine method and 99.1% for the 100 $\mu$L urine method. However, the corresponding sensitivity, defined as the percent of those samples classified as positive (based on serum HIV testing) that tested positive in the urine assay, was unacceptable at 73.6%. In contrast, the method of the present invention has a consistent sensitivity of about 90%.

5

## Table 1

| Sample Type | Sample Number | Mean OD | SD | Specificity (5SD) | Sensitivity (S/N) |
|---|---|---|---|---|---|
| **100 µL diluted*** | | | | | |
| Negative | 161 | 0.013 | 0.0098 | 98.8 | 97.5 |
| Positive | 319 | 0.903 | 0.7705 | 86.5 | 90.9 |
| **400 µL**** | | | | | |
| Negative | 140 | 0.025 | 0.0158 | 97.9 | 93.6 |
| Positive | 270 | 0.587 | 0.4870 | 88.5 | 93.7 |
| **100 µL***** | | | | | |
| Negative | 115 | 0.063 | 0.0583 | 99.1 | 15.7 |
| Positive | 250 | 0.723 | 0.5601 | 73.6 | 99.6 |

*a mixture of affinity-purified envelope and core viral proteins and whole viral lysate

**recombinant envelope and core proteins

***whole viral lysate

In order to distinguish between negative and reactive test results, it is desirable to have a large separation between the negative mean absorbance and the positive mean absorbance. As shown in Table 1, the method of the present invention produced a mean absorbance of the negative population (0.013) which was 70-fold lower than the mean absorbance of the reactive population (0.903). In contrast, using the 400 µL urine method, the mean absorbance of the negative population (0.025) was only 24-fold lower than the mean absorbance of the reactive population (0.587). Using the 100 µL urine method, the mean absorbance of the negative population (0.063) was only 12-fold lower than the mean absorbance of the reactive population (0.560). Thus, Table 1 clearly illustrates that the method of the present invention produced an increased separation between the negative and reactive sample populations, in contrast to the prior art methods using undiluted urine.

EXAMPLE 2

HIV Antibody Detection of Clinical Samples

Serum and urine samples were obtained from individuals with renal dysfunction. One hundred thirteen (113) samples were tested and shown to be HIV-1 antibody reactive by serum testing (Abbott Laboratories). The urine samples were tested using the method of the present invention, as previously described, to determine the presence of anti-HIV antibodies in urine. Urine samples were diluted at a ratio of 100µL urine to 100 µL diluent (the "100 µL diluted urine assay") and 200 µL urine to 100 µL diluent (the "200 µL diluted urine assay"). Reactivity was determined by S/N ratio.

Of the 113 samples classified as positive by serum testing, the 100 µL diluted urine assay and the 200 µL diluted urine assay, respectively, determined 101 samples (89.4%) and 105 samples (92.9%) to be positive.

Based on interviews with the individuals who provided the samples, 52 individuals (46%) appeared to be associated with high risk activities for contacting AIDS. These activities were classified as intravenous drug abuser (IVDA), prostitute, homosexual, multiple sex partners, factor VIII recipient, and multiple transfusion recipient. If any individuals were associated with more than one of the above high risk activities, they were also classified as multiple risk.

As shown in Table 2, the method of the present invention established as reactive from 100% to 45.5% of urine samples from high risk individuals. Thus, the method of the present invention is an acceptable method for detecting the presence of anti-HIV antibodies in urine samples.

### Table 2

| Risk Group Category | Number | (%) | Reactive* | (%) |
|---|---|---|---|---|
| IVDA | 25 | (47.2) | 21 | (84.0) |
| Prostitute | 11 | (20.8) | 5 | (45.5) |
| Homosexual | 9 | (17.0) | 8 | (88.9) |
| Multiple sex partners | 5 | (9.4) | 2 | (40.0) |
| Factor VIII recipient | 1 | (1.9) | 1 | (100.0) |
| Multiple transfusions | 1 | (1.9) | 1 | (100.0) |
| Multiple risk groups | 10 | (18.9) | 7 | (70.0) |

*Confirmed by urine western blot testing at a 1:1 dilution. Bands representing the three gene products env, pol and gag were present.

## Claims

1. A method for detecting anti-HIV antibodies which comprises the steps of:
   a) collecting a urine sample from an individual;
   b) diluting said urine sample with a diluent; and
   c) assaying said diluted sample for the presence of said antibodies.

2. The method of claim 1 wherein said assaying step further comprises reacting said diluted sample with viral HIV antigens bound to a solid phase.

3. The method of claim 2 wherein said HIV antigens comprise a mixture of HIV-1 viral lysate and HIV-1 envelope and core proteins.

4. The method of claim 1 wherein said assaying step further comprises reacting said diluted sample with recombinant HIV antigens bound to a solid phase.

5. The method of claim 1 wherein the ratio of urine sample to diluent is about 4:1 to 1:4.

6. The method of claim 4 wherein the ratio of urine sample to diluent is about 1:1.